# EUROPEAN PATENT APPLICATION

(11) **EP 4 458 389 A1**
(43) Date of publication of application: **06.11.2024**
(21) Application number: 22920407.8
(22) Date of filing: 20.10.2022
(51) Int. Cl.: A61M 5/315

(54) **GASKET FOR SYRINGE AND SYRINGE PROVIDED WITH SAME**

(30) Priority: 17.01.2022 JP 2022005317
(71) Applicant: Terumo Kabushiki Kaisha, Tokyo 151-0072 (JP)
(72) Inventor: WATANABE, Yuusuke, Fujinomiya-shi, Shizuoka 418-0004 (JP); HASEGAWA, Tatsuhiko, Fujinomiya-shi, Shizuoka 418-0004 (JP)
(74) Representative: Casalonga
(86) International application number: PCT/JP2022/039164
(87) International publication number: WO 2023/135884

(57) **Abstract**

A gasket is formed to be in liquid-tight and slidable contact with the inside of a plastic outer cylinder of a syringe, the gasket includes: a gasket body including an elastic body; and a coating layer provided at least at a part in contact with the syringe, the coating layer is an elastic cured coating layer containing a silicone-based resin as a main component and containing a carbodiimide compound together with a silane coupling agent, and the silicone-based resin includes a condensate of a reactive silicone having a silanol group at both terminals and has a siloxane bond derived from the silanol group.

## Description

### BACKGROUND

### Technical Field

The present invention relates to a gasket, for use in a syringe, having stable sliding performance, and a syringe including a gasket.

### Related Art

A prefilled syringe, which is filled with a drug solution in advance, has been conventionally used for reasons such as prevention of mistake in use of a drug, prevention of hospital infection, disposability, and efficiency in hospital service. Syringes including a syringe to be used as a prefilled syringe generally include an outer cylinder, a gasket slidable inside the syringe, and a plunger that operates movement of the gasket. In many syringes, a silicone oil or the like is applied as a lubricant to the sliding portion of the gasket outer surface or the syringe inner surface in order to enhance the sliding performance of the gasket and obtain a high degree of flow accuracy without generating a large irregularity in discharge of a drug solution. However, it is known that an interaction may occur between a certain kind of drug solution and the lubricant such as a silicone oil. If the drug solution is stored for a long time after filling of a syringe with the drug solution, the drug solution is denatured by the interaction, and thus such a drug solution is difficult to use in a prefilled syringe.

There is a demand for a prefilled syringe that can maintain the stability of a drug solution without a lubricant while stored for a long time in a state of being filled with the drug solution.

To solve the above-described problem, as disclosed in Patent Document 1 (JP S62-32970 A) and Patent Document 2 (JP 2002-089717 A: U.S. Patent No. 7111848), prefilled syringes have been proposed in which the surface of a gasket is covered with a fluorine-based resin that is a material having a lower friction coefficient than the material of the gasket body to eliminate use of a lubricant.

The present applicant has proposed a gasket having a coating layer including a fluorine-based resin, a silicon-based resin, and a urethane-based resin as disclosed in Patent Document 3 (JP 2004-321614 A) and a gasket having a coating layer that includes a film formed using a composition containing a sliding performance-imparting component and a flexibility-imparting component and includes fine particles held by the film to form a rough surface on the gasket as disclosed in Patent Document 4 (JP 2006-167110 A).

The present applicant has proposed a gasket formed so as to be in liquid-tight and slidable contact with the inside of an outer cylinder of a syringe, and the gasket includes a gasket body including an elastic body and includes a coating layer provided at least at a part in contact with the syringe, the coating layer includes a composition containing a silicone-based resin and does not contain solid fine particles, and the silicone-based resin includes a condensate of a reactive silicone having a terminal silanol group and has a siloxane bond derived from a silanol group (Patent Document 5: WO 2009/084646).

### Citation List

### Patent Documents

Patent Document 1: JP S62-32970 A
Patent Document 2: JP 2002-089717 A (U.S. Patent No. 7111848, US 2004-084852 A, US 2005-212222 A, EP 1317937 A, WO 02-22192)
Patent Document 3: JP 2004-321614 A
Patent Document 4: JP 2006-167110 A
Patent Document 5: WO 2009/084646 (US 2010-324501 A, US 2015-133873 A, EP 2226088 A)

### SUMMARY

The gaskets disclosed in Patent Document 1 (JP S62-32970 A) and Patent Document 2 (JP 2002-089717 A, U.S. Patent No. 7111848) are expected to be effective under some use conditions. But in a preparation for a prefilled syringe demanded to discharge a drug solution under a high pressure and have performance of stably discharging a drug solution little by little with a very high accuracy for a long time by using a syringe pump or the like, the liquid-tightness and the sliding performance, which are fundamental performance demanded for the syringe, are still in a trade-off relationship. A syringe is needed that achieves both of these performances at a high level and further has a high performance.

That is, in administration of a drug solution by using a syringe pump, when the drug solution is discharged in a condition where the flow rate is so low that the flow is invisible (for example, in a syringe having a diameter of about 24 mm, the moving speed at a discharge speed of 1 mL/hour is about 2 mm/hour), an unstable discharge state called pulsation is liable to occur and thus accurate administration of the drug solution may be prevented.

The gaskets disclosed in Patent Document 3 (JP 2004-321614 A) and Patent Document 4 (JP 2006-167110 A) are liquid-tight and have stable sliding performance without applying a lubricant to the sliding surface. But in the former, various materials are used to form the coating layer to cause problems in terms of production and cost. The latter has a problem that the fine particles held by the coating layer cause the formation of the coating layer to be difficult.

The gasket disclosed in Patent Document 5 (WO 2009/084646) is liquid-tight and has stable sliding performance without applying a lubricant to the sliding surface. However, the inventor of the present application has found that stronger coating is desirable in the case of a syringe system used under a higher pressure or a syringe use in which the syringe is slid repeatedly.

Therefore, the present invention solves the above-described problems, and provides a gasket that has stable sliding performance without applying a lubricant to the sliding surface and includes an applied film that is extremely less likely to peel off, and a syringe including the gasket.

An object described above is achieved by the following.

A gasket for use in a syringe, the gasket formed to be in liquid-tight and slidable contact with an inside of a plastic outer cylinder of a syringe, the gasket including: a gasket body including an elastic body; and a coating layer provided at least at a part in contact with the syringe, the coating layer being an elastic cured coating layer containing a silicone-based resin as a main component and containing a carbodiimide compound together with a silane coupling agent, the silicone-based resin that includes a condensate of a reactive silicone having a silanol group at both terminals and has a siloxane bond derived from the silanol group.

Furthermore, an object described above is achieved by the following. A syringe including: a plastic outer cylinder; the above-described gasket slidably accommodated in the plastic outer cylinder; and a plunger attached to or attachable to the gasket.

### BRIEF DESCRIPTION OF DRAWINGS

FIG. 1 is a front view of a gasket as an example of the present invention;
FIG. 2 is a sectional view of the gasket shown in FIG. 1;
FIG. 3 is a plan view of the gasket shown in FIG. 1;
FIG. 4 is a bottom view of the gasket shown in FIG. 1;
FIG. 5 is a sectional view of a prefilled syringe in which the gasket shown in FIG. 1 is used;
FIG. 6 is a photograph showing a result of an experiment in which a gasket as an example of the present invention is used; and
FIG. 7 is a photograph showing a result of an experiment in which a gasket as a comparative example is used.

### DETAILED DESCRIPTION

A gasket as an example of the present invention will be described.

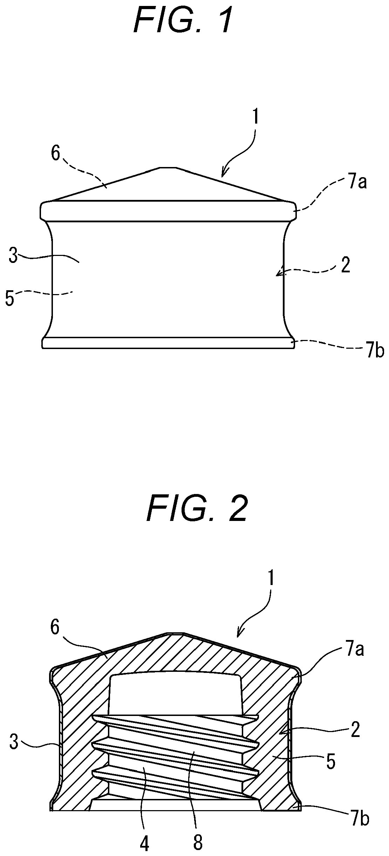

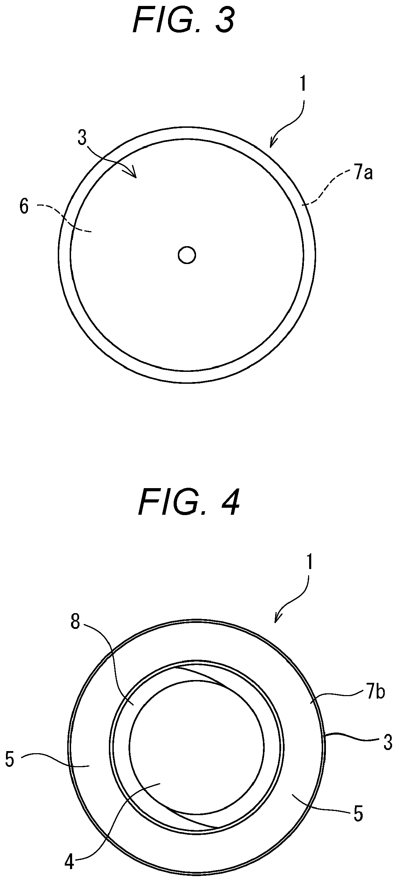

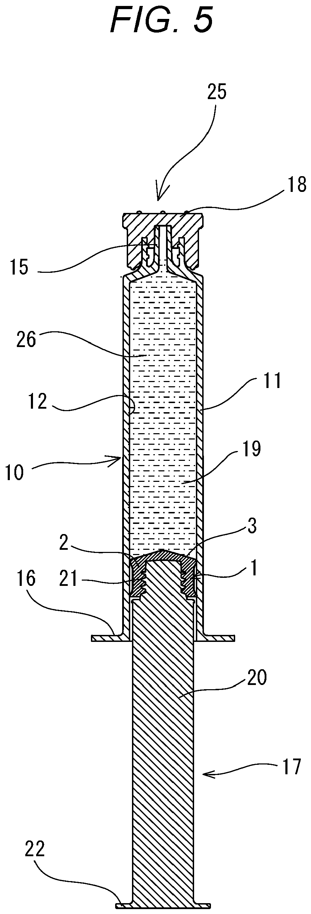

FIG. 1 is a front view of a gasket as an example of the present invention. FIG. 2 is a sectional view of the gasket shown in FIG. 1. FIG. 3 is a plan view of the gasket shown in FIG. 1. FIG. 4 is a bottom view of the gasket shown in FIG. 1. FIG. 5 is a sectional view of a prefilled syringe in which the gasket shown in FIG. 1 is used.

A gasket 1 of the present invention is a gasket formed to be in liquid-tight and slidable contact with the inside of a plastic outer cylinder of a syringe. The gasket 1 includes a gasket body (core) 2 including an elastic body and includes a coating layer 3 provided at least at a part in contact with the syringe, the coating layer 3 is an elastic cured coating layer containing a silicone-based resin as a main component and containing a carbodiimide compound together with a silane coupling agent, and the silicone-based resin includes a condensate of a reactive silicone having a silanol group at both terminals and has a siloxane bond derived from the silanol group.

The gasket of the present invention will be described with reference to an example.

A gasket 1 of this example is a gasket 1 for use in a syringe, and is liquid-tightly and slidably accommodated in an outer cylinder 11 for use in a syringe. The gasket 1 includes a coating layer 3 provided at a part in contact with the outer cylinder 11. The gasket 1 includes a gasket body (core) 2 and the coating layer 3 provided at least at a part that is the outer surface of the gasket body 2 in contact with the outer cylinder inner surface. The coating layer 3 may be provided on the entire outer surface of the gasket body 2.

As shown in FIGS. 1, 2, and 5, the gasket body 2 of the gasket 1 for use in a syringe includes a main body 5 extending in substantially the same outer diameter, a tapered portion 6 provided on the distal side of the main body 5 and tapered toward the distal side, a plunger attachment portion 4 provided inside from the base of the main body 5 toward the distal side, a distal side annular rib 7a provided on the side surface of the distal portion of the main body 5, and a rear end side annular rib 7b provided on the side surface of the rear end portion of the main body 5.

As shown in FIGS. 2 and 4, the plunger attachment portion 4 is a substantially columnar recess extending inside the main body 5 from the base to the vicinity of the distal portion, and the side surface of the recess is provided with a screw portion 8 that can be screwed with a screw portion formed in the distal portion of a plunger. The distal end surface of the recess is formed to be substantially flat. The plunger attachment portion is not limited to the screw portion, and may be an engagement portion that engages with the distal portion of a plunger.

The annular ribs 7a and 7b are produced to be slightly larger than the inner diameter of the outer cylinder 11 for use in a syringe, and therefore in the outer cylinder 11, the annular ribs 7a and 7b are compressed and deformed. In this example, two annular ribs are provided, but one or three or more annular ribs may be provided.

The constituent material of the gasket body (core) 2 is preferably an elastic material. The elastic material is not particularly limited, and examples of the elastic material include various rubber materials (in particular, those subjected to vulcanization treatment) such as natural rubber, isoprene rubber, butyl rubber, chloroprene rubber, nitrile-butadiene rubber, styrene-butadiene rubber, and silicone rubber, styrene-based elastomers, hydrogenated styrene-based elastomers, and styrene-based elastomers mixed with a polyolefin such as polyethylene, polypropylene, polybutene, or an α-olefin copolymer, an oil such as liquid paraffin or a process oil, or a powder inorganic substance such as talc, cast, or mica. Furthermore, a polyvinyl chloride-based elastomer, an olefin-based elastomer, a polyester-based elastomer, a polyamide-based elastomer, a polyurethane-based elastomer, a mixture thereof, or the like can be used as the constituent material. The constituent material is particularly preferably diene-based rubber or a styrene-based elastomer from the viewpoint of having elastic characteristics and enabling γ-ray sterilization, electron beam sterilization, and high-pressure steam sterilization.

The coating layer 3 is to be provided at least at the annular rib part. Specifically, the coating layer 3 is to be provided at the part of the distal side annular rib 7a and the rear end side annular rib 7b. The coating layer 3 preferably has a thickness of 1 to 30 µm, and particularly preferably 3 to 10 µm. If the thickness is 1 µm or more, the required sliding performance is exhibited, and if the thickness is 30 µm or less, the elasticity of the gasket is not affected. As the silicone-based resin, either a solvent-based resin dissolved in an organic solvent or an aqueous resin emulsified and dispersed in water can be applied, but an aqueous resin is preferable from the viewpoint of the influence on the gasket material or the suitability as a drug solution storage container. The coating layer 3 includes a resin including a material having a lower friction coefficient than the elastic material included in the gasket body 1.

The coating layer 3 is an elastic cured coating layer containing a silicone-based resin as a main component and containing a carbodiimide compound together with a silane coupling agent, and the silicone-based resin includes a condensate of a reactive silicone having a silanol group at both terminals and has a siloxane bond derived from the silanol group.

The composition containing the reactive silicone-based resin is preferably a thermosetting silicone-based resin or a room-temperature curable silicone-based resin, and particularly preferably a thermosetting silicone-based resin from the viewpoint of workability, and the like.

The reactive silicone used has a silanol group at both terminals. In a case where a polysiloxane-based silicone having a silanol group at both terminals as described above is used as the reactive silicone, the condensate of the reactive silicone has a siloxane bond in the entire main chain.

As the reactive silicone having a silanol group at both terminals, specifically, a polysiloxane-based silicone having a silanol group at both terminals is suitable such as both terminals silanol polydimethylsiloxane, both terminals silanol polydiphenylsiloxane, or both terminals silanol diphenylsiloxane-dimethylsiloxane copolymer. The form of the reactive silicone is not particularly limited, and it is also possible to use a product obtained by dispersing, emulsifying, and dissolving a polysiloxane including a reactive silicone siloxane compound as described above or its condensate in an aqueous medium, a copolymer emulsion obtained by copolymerizing an alkoxysilyl group-containing vinyl monomer with another vinyl monomer as necessary, an emulsion obtained by complexing a polysiloxane with an organic polymer, and the like.

The resin composition forming the coating layer 3 contains a silane coupling agent. In other words, the elastic cured coating layer 3 contains a silane coupling agent.

The silane coupling agent is preferably at least one selected from an alkylalkoxysilane, a phenylalkoxysilane, an aminoalkylalkoxysilane, and a glycidoxyalkylalkoxysilane.

The resin composition forming the coating layer 3 preferably contains an alkylalkoxysilane or a phenylalkoxysilane as a first silane coupling agent, and further contains an aminoalkylalkoxysilane and/or a glycidoxyalkylalkoxysilane as a second silane coupling agent.

Furthermore, the resin composition forming the coating layer 3 preferably contains an alkylalkoxysilane or a phenylalkoxysilane as a first silane coupling agent, and further contains an aminoalkylalkoxysilane as a second silane coupling agent and a glycidoxyalkylalkoxysilane as a third silane coupling agent.

The alkylalkoxysilane has at least one alkyl group having 1 to 20 carbon atoms and at least one alkoxy group having 1 to 4 carbon atoms. Examples of the suitable alkylalkoxysilane include methyltrimethoxysilane, methyltriethoxysilane, methyltriisobutoxysilane, methyltributoxysilane, methyl sec-trioctyloxysilane, isobutyltrimethoxysilane, cyclohexylmethyldimethoxysilane, diisopropyldimethoxysilane, propyltrimethoxysilane, diisobutyldimethoxysilane, n-octylmethoxysiloxane, ethyltrimethoxysilane, dimethyldimethoxysilane, octyltriethoxysilane, hexyltrimethoxysilane, hexyltriethoxysilane, octamethylcyclotetrasiloxane, methyltri(acryloyloxyethoxy)silane, octyltriethoxysilane, lauryltriethoxysilane, stearyltrimethoxysilane, stearyltriethoxysilane, ethyltriethoxysilane, propyltriethoxysilane, butyltrimethoxysilane, butyltriethoxysilane, pentyltrimethoxysilane, pentyltriethoxysilane, heptyltrimethoxysilane, heptyltriethoxysilane, octyltrimethoxysilane, nonyltrimethoxysilane, nonyltriethoxysilane, decyltrimethoxysilane, decyltriethoxysilane, undecyltrimethoxysilane, undecyltriethoxysilane, dodecyltrimethoxysilane, dodecyltriethoxysilane, tridecyltrimethoxysilane, tridecyltriethoxysilane, tetradecyltrimethoxysilane, tetradecyltriethoxysilane, pentadecyltrimethoxysilane, pentadecyltriethoxysilane, hexadecyltrimethoxysilane, hexadecyltriethoxysilane, heptadecyltrimethoxysilane, heptadecyltriethoxysilane, octadecyltrimethoxysilane, octadecyltriethoxysilane, nonadecyltrimethoxysilane, nonadecyltriethoxysilane, eicosyltrimethoxysilane, and eicosyltriethoxysilane.

Suitable examples of the alkylphenoxysilane include methyltriphenoxysilane. Suitable phenoxyalkoxysilanes include phenyltrimethoxysilane, phenyltriethoxysilane, diphenyldimethoxysilane, and diphenyldiethoxysilane.
Furthermore, usable alkylalkoxysilanes include methyltri(glycidyloxy)silane, trimethylchlorosilane, dimethylchlorosilane, methyltrichlorosilane, tetraethoxysilane, heptadecafluorodecyltrimethoxysilane, tridecafluorooctyltrimethoxysilane, and tetrapropoxysilane.

Suitable aminoalkylalkoxysilanes include 3-aminopropyltriethoxysilane, 3-(2-aminoethyl)aminopropyltrimethoxysilane, 3-(2-aminoethyl)aminopropylmethyldimethoxysilane, 3-aminopropyltrimethoxysilane, and 3-phenylaminopropyltrimethoxysilane.

Suitable glycidoxyalkylalkoxysilanes include 3-glycidoxypropyltrimethoxysilane, 3-glycidoxypropyltriethoxysilane, 3-glycidoxypropylmethyldiethoxysilane, 3-glycidoxypropylmethyldimethoxysilane, and 2-(3,4-epoxycyclohexyl)ethyltrimethoxysilane.
Furthermore, as the silane coupling agent, a silane-based compound may be used such as 3-ureidopropyltriethoxysilane, diallyldimethylsilane, n-octyldimethylchlorosilane, tetraethoxysilane, or trifluoropropyltrimethoxysilane.

The composition forming the coating layer 3 may contain the first and the second silane coupling agents. The first silane coupling agent is preferably selected from an alkylalkoxysilane, an alkylphenoxysilane, and a phenylalkoxysilane. As the second coupling agent, an aminoalkylalkoxysilane or a glycidoxyalkylalkoxysilane is preferably used. The composition forming the coating layer may contain the first, the second, and the third silane coupling agents. The first silane coupling agent is preferably selected from an alkylalkoxysilane, an alkylphenoxysilane, and a phenylalkoxysilane. The second silane coupling agent is preferably an aminoalkylalkoxysilane, and as the third silane coupling agent, a glycidoxyalkylalkoxysilane is preferably used.

The resin composition forming the coating layer 3 contains a carbodiimide compound. In other words, the elastic cured coating layer 3 contains a carbodiimide compound.

In the resin composition forming the elastic cured coating layer 3, the silicone-based resin or the silane coupling agent has a functional group that reacts with a carbodiimide group of the carbodiimide compound. In the resin composition forming the elastic cured coating layer 3, the silicone-based resin and the silane coupling agent may have a functional group that reacts with a carbodiimide group of the carbodiimide compound.

The functional group that reacts with a carbodiimide group is preferably at least one selected from a carboxyl group, a hydroxyl group, an amino group, an amide group, a sulfonate group, and a phosphate group.

The carbodiimide compound is preferably a compound having two or more carbodiimide groups (-N=C=N-). The carbodiimide group functions as a crosslinkable functional group. Furthermore, the carbodiimide compound may have one or more isocyanate groups derived from a polyisocyanate (Q) as a raw material, in addition to the carbodiimide group.

In the elastic cured coating layer 3, the functional group that reacts with a carbodiimide group in the silicone-based resin and/or the silane coupling agent is bonded to a carbodiimide group of the carbodiimide compound. In particular, a carbodiimide compound having two or more carbodiimide groups is bonded to the silicone-based resin and/or the silane coupling agent at two positions to form a strong coating layer.

The carbodiimide compound is preferably a polycarbodiimide or a cyclic carbodiimide compound, and particularly preferably a polycarbodiimide. Furthermore, an aliphatic polycarbodiimide compound is preferable. The polycarbodiimide preferably has a number average molecular weight of 500 to 20000.

Examples of the polycarbodiimide compound include aliphatic polycarbodiimides such as polyhexamethylene carbodiimide, polytrimethylhexamethylene carbodiimide, polycyclohexylene carbodiimide, poly(methylene biscyclohexylene carbodiimide), and poly(isophorone carbodiimide), aromatic polycarbodiimides such as poly(phenylene carbodiimide), poly(naphthylene carbodiimide), poly(tolylene carbodiimide), poly(methyl diisopropylphenylene carbodiimide), poly(triethylphenylene carbodiimide), poly(diethylphenylene carbodiimide), poly(triisopropylphenylene carbodiimide), poly(diisopropylphenylene carbodiimide), poly(xylylene carbodiimide), poly(tetramethylxylylene carbodiimide), poly(methylenediphenylene carbodiimide), and poly[methylenebis(methylphenylene)carbodiimide], poly(4,4'-dicyclohexylmethane carbodiimide), poly(N,N'-di-2,6-diisopropylphenyl carbodiimide), poly(1,3,5-triisopropylphenylene-2,4-carbodiimide), urethane condensates of polyoxyalkylene alkyl (or alkenyl) (C = 8 to 24) ethers and polyoxyalkylene (n = 1 to 300) glycols with hexamethylene diisocyanate [or tolylene diisocyanate, xylylene diisocyanate, bis(isocyanatophenyl)methane], condensates of lauryl alcohol, hexamethylene diisocyanate, and tolylene diisocyanate, alkyl-substituted carbodiimide compounds, urethane condensates of dioxyethylene stearyl ether and a polyethylene glycol (n = 5) with hexamethylene diisocyanate, urethane condensates of tetraoxyethylene stearyl ether and polyethylene glycols with hexamethylene diisocyanate, and urethane condensates of dioxyethylene lauryl ether and a polyethylene glycol (n = 5) with diphenylmethane diisocyanate.

Examples of the carbodiimide compound include water-soluble compounds, compounds that form an emulsion in water, and compounds that form a dispersion in water. Any of these compounds may be used, and a plurality of the compounds may be mixed and used. In the present invention, the carbodiimide compound is preferably a water-soluble carbodiimide compound. The polycarbodiimide may terminate in a water-soluble polymer.

The content of the carbodiimide compound in the resin composition forming the coating layer 3 is preferably 0.01 to 0.2 parts by weight, and particularly preferably 0.01 to 0.05 parts by weight.

The gasket 1 of the present invention having the coating layer 3 as described above has stable sliding performance without applying a lubricant to the sliding surface, and can maintain the sealing state in the drug storage space. Thus, the covering layer has a sufficient strength, and therefore the coating layer is not peeled off or partially detached from the gasket body 2.

The elastic cured coating layer 3 is preferably a thermosetting elastic cured coating layer. The coating layer preferably has a thickness of 1 to 30 µm, and particularly preferably 3 to 10 µm.

In the coating layer, the initial sliding resistance value is preferably equal to or less than the maximum dynamic sliding resistance value. Furthermore, the dynamic sliding resistance value at the time of low-speed sliding (100 mm/min) of the gasket in the outer cylinder is preferably 20 N or less for a syringe having a volume of 1 to 20 mL, 100 N or less for a syringe having a volume of more than 20 mL and less than 100 mL, and 150 N or less for a syringe having a volume of 100 mL or more.

Next, a method of forming a coating layer 3 will be described. In the method of forming a coating layer, a coating liquid is prepared by dispersing and suspending a mixture obtained by blending the silicone-based resin and the carbodiimide compound described above in required amounts at a required composition in purified water. Then, the coating liquid is applied to a clean gasket surface and then cured to obtain a coating layer. At this time, the coating liquid can be applied to the gasket surface with a method known in related art, such as an immersion method or a spraying method. The coating liquid is particularly preferably applied by spraying (spray applied) in a state where the object to be coated is rotated (specifically, at 100 to 600 rpm). Furthermore, the spray application is preferably performed after the portion to be coated in the gasket is heat-treated to about 60 to 120 degrees C. In this way, the coating liquid is not repelled and quickly fixed to the surface to be coated.

The curing method may be a method of leaving at room temperature, but thermal curing is preferable. The thermal curing method is not particularly limited as long as it does not alter or deform a gasket base material, and examples of the method include hot air drying and drying with a drying furnace using infrared rays. Alternatively, a method known in related art can be used such as a method in which a vacuum dryer is used. The coating layer formed is to have a thickness of about 1 to 30 µm, and preferably 3 to 10 µm. Such a coating layer can be easily formed by appropriately controlling the concentration of the mixed liquid, the immersion method, or the spraying method.

In preparation of the coating liquid containing the silicone-based resin, a catalyst capable of promoting thermal curing may be used as an additive.
As the catalyst, an acid, an alkali, an amine, an organic salt of a metal, a titanate, or a borate is used, and zinc octylate, iron octylate, or an organic acid salt of cobalt, tin, lead, or the like is preferable.

In particular, usable organic acid salts of tin include bis(2-ethylhexanoate)tin, bis(neodecanoate)tin, di-n-butylbis(2-ethylhexylmalate)tin, di-n-butylbis(2,4-pentanedionate)tin, di-n-butylbutoxychlorotin, di-n-butyldiacetoxytin, di-n-butyldilaurate tin, dimethyldineodecanoate tin, dimethylhydroxy(oleate)tin, and tin dioctyldilaurate.

In preparation of the coating liquid containing the silicone-based resin, a surfactant or an additive such as an alcohol may be used for uniform emulsification, suspension, and dispersion in the liquid.

The surfactant is preferably an anionic (anion) surfactant. Any anionic (anion) surfactant may be used, and usable anionic (anion) surfactants include aliphatic monocarboxylates, polyoxyethylene alkyl ether carboxylates, N-acylsarcosinates, N-acylglutamates, dialkylsulfosuccinates, alkanesulfonates, alpha-olefin sulfonates, linear alkylbenzenesulfonates, molecular chain alkylbenzenesulfonates, naphthalene sulfonate-formaldehyde condensates, alkylnaphthalenesulfonates, N-methyl-N-acyltaurine, alkyl sulfates, polyoxyethylene alkyl ether sulfates, fat sulfate salts, alkyl phosphates, polyoxyethylene alkyl ether sulfates, and polyoxyethylene alkyl phenyl ether sulfates.

A nonionic (nonion) surfactant may also be used. Any nonionic (nonion) surfactant may be used, and usable nonionic (nonion) surfactants include polyoxyethylene alkyl ethers, polyoxyalkylene derivatives, polyoxyethylene alkyl phenyl ethers, polyoxyethylene sorbitan fatty acid esters, fatty acid alkanolamides, glycerin fatty acid esters, sorbitan fatty acid esters, polyoxyethylene alkylamines, and alkyl alkanolamides.

A syringe 10 of the present invention includes an outer cylinder 11, a gasket 1 slidably accommodated in the outer cylinder 11, and a plunger 17 attached to or attachable to the gasket 1.

Specifically, as shown in FIG. 5, the syringe 10 includes the outer cylinder 11, for use in a syringe, having a distal portion provided with a needle attachment portion 15 and having a rear end portion provided with a flange 16 facing the rear end portion, the gasket 1, for use in a syringe, slidable liquid-tightly and airtightly with an inner surface 12 of the outer cylinder 11 for use in a syringe, the plunger 17 attached to or attachable to the gasket 1 for use in a syringe, a sealing member 18 that seals the needle attachment portion 15 of the outer cylinder 11 for use in a syringe, and a drug storage portion 19 formed to be surrounded by the sealing member 18, the inner surface 12 of the outer cylinder, and the gasket 1 for use in a syringe to store a drug 26.

Not the sealing member 18 but a needle may be attached to the needle attachment portion 15. The sealing member may be a member having a puncture portion into which a double-headed needle can be directly inserted as shown in FIG. 5, or may be a member such that a drug can be discharged only by removing the sealing member. The gasket 1 includes the above-described coating layer 3. In the syringe 10, the dynamic sliding resistance value at the time of low-speed sliding (100 mm/min) of the gasket 1 in the outer cylinder 11 is preferably 20 N or less for a syringe having a volume of 1 to 20 mL, and 150 N or less for a syringe having a volume of 100 mL or more. Such a low dynamic sliding resistance value can be obtained by the gasket 1 having the above-described coating layer 3.

This medical device is particularly a prefilled syringe 25, and includes the syringe 10 and the drug 26 as shown in FIG. 5.

The outer cylinder 11 for use in a syringe is a cylindrical member having a distal portion provided with the needle attachment portion 15 and having a rear end portion provided with the flange 16. The outer cylinder 11 for use in a syringe is formed using a transparent or translucent material. The outer cylinder 11 is preferably formed using a material having a low oxygen permeability and a low water vapor permeability. The forming material is preferably a material having a glass transition point or a melting point of 110 degrees C or higher.

As the material forming the outer cylinder 11, widely used various rigid plastic materials are preferable such as polyolefins such as polypropylene, polyethylene, poly(4-methylpentene-1), and a cyclic polyolefin, polyesters such as polyethylene terephthalate, polyethylene naphthalate, and amorphous polyarylate, polystyrene, polyamides, polycarbonate, polyvinyl chloride, acrylic resins, an acrylonitrile-butadiene-styrene copolymer, and amorphous polyetherimide, and in particular, polypropylene, poly(4-methylpentene-1), a cyclic polyolefin, polyethylene naphthalate, and amorphous polyetherimide are preferable from the viewpoint of transparency and heat sterilization resistance. These resins can be used not only in the outer cylinder for use in a syringe, but can be commonly used in containers capable of storing a drug. Furthermore, glass may be used as the forming material.

As shown in FIG. 5, the plunger 17 includes a main body 20 having a cross-shaped section and extending in the axial direction, a plunger side screw portion 21 to be screwed with a plunger attachment portion 4 and provided at a distal portion of the plunger 17, a disk-shaped gasket pressing portion provided between the plunger side screw portion 21 and the main body 20, a pressing disk portion 22 provided at a rear end of the main body 20, and a disk-shaped rib provided midway on the main body 20.

Then, the drug 26 is stored inside the syringe 10 of this example. The drug 26 may be a liquid drug or a solid drug such as a powdered drug or a freeze-dried drug, but in a case where a drug solution having low water solubility and high adsorptivity, a drug solution containing a surfactant and having low viscosity and high permeability, or the like is stored, a silicone oil is not required, and in a case where the coating layer 3 is provided at a part in contact with the stored drug, drug adsorption or the like can be prevented, and thus the drug can be more suitably used.

As the constituent materials of the plunger 17 and the sealing member 18, rigid or semi-rigid resins such as polyvinyl chloride, high density polyethylene, polypropylene, polystyrene, polyethylene terephthalate, polycarbonate, and acrylic resins are preferably used.

### Examples

Hereinafter, specific examples of the present invention will be described.

### (Example 1)

A core (gasket core member) of a gasket, for use in a 100 mL syringe, having the shape shown in FIGS. 1 and 2 was produced using butyl rubber. The core was formed by press-molding a vulcanizable rubber composition obtained by blending an additive to butyl rubber. The obtained core has a shape having a length of 18 mm, an outer diameter of 33 mm at the distal side part and the rear end side annular rib part, an outer diameter of 32 mm at the part having the same outer diameter between the distal side annular rib and the rear end side annular rib, a length (depth) of the recess for plunger attachment having the female screw portion inside of 10 mm, an inner diameter of 20 mm at the distal side of the recess for plunger attachment, and an inner diameter of 23 mm at the rear end side.

### A1: Silicone-based resin

1) 1501 Fluid (product name) (manufactured by Dow Corning Toray Co., Ltd.) containing both terminals silanol polydimethylsiloxane as main component: 25 parts by weight
2) Z-6366 (product name) (manufactured by Dow Corning Toray Co., Ltd.) containing methyltrimethoxysilane as main component: 0.1 parts by weight
3) Mixture of Z-6011 (product name) (manufactured by Dow Corning Toray Co., Ltd.) containing 3-aminopropyltriethoxysilane as main component with ethanol solution of maleic anhydride: 1 part by weight (resin ratio: 50%)
4) Z-6040 (product name) (manufactured by Dow Corning Toray Co., Ltd.) containing 3-glycidoxypropyltrimethoxysilane as main component: 0.5 parts by weight

### B: Carbodiimide compound

Aqueous solution of polyvalent carbodiimide [polyvalent carbodiimide compound content: 40 wt%, product name: CARBODILITE V-02, manufactured by Nisshinbo Chemical Inc., pH: 9 to 12, viscosity (representative value): 100 mPa s, NCN equivalent (chemical formula weight per mol of carbodiimide group): 590, aqueous solution of hydrophilic group-modified polycarbodiimide compound]

### C: Preparation of coating liquid

A silicone-based resin mixture was produced by mixing 10 parts by weight of linear sodium alkylbenzene sulfonate with 100 parts by weight of the silicone-based resin (A1) including 1) to 4) described above. To 66 parts by weight of purified water, 29 parts by weight of the silicone-based resin mixture and 1 part by weight of tin dioctyldilaurate were added to prepare a main agent. To 100 parts by weight of the main agent, 5 parts by weight of the carbodiimide compound (B: mixture of the polyvalent carbodiimide compound and water) was added, and the resulting mixture was mixed and stirred to prepare a liquid for coating.

Then, the gasket core member produced as described above was heat-treated at 100 degrees C for 15 minutes under a room temperature and normal pressure environment and then rotated (at 300 rpm) around its central axis, and the liquid for coating having the above-described composition was spray-applied (in an amount of 0.2 mL) from the rotating side surface side of the gasket, and then dried at 100 degrees C for 15 minutes to produce a gasket of the present invention. Thereafter, washing was performed with purified water at 80 degrees C or higher in order to wash off the excess coating liquid on the produced gasket. The coating layer formed on the surface of the core member had an average thickness of about 8 µm. This gasket was used in Example 1.

### (Example 2)

A liquid for coating was prepared in the same manner as in Example 1 except that 1 part by weight of the carbodiimide compound (B: mixture of the polyvalent carbodiimide compound and water) was added to 100 parts by weight of the main agent in Example 1 and the resulting mixture was mixed and stirred to prepare a liquid for coating.

Then, a gasket core member produced as described above was heat-treated at 100 degrees C for 15 minutes under a room temperature and normal pressure environment and then rotated (at 300 rpm) around its central axis, and the liquid for coating having the above-described composition was spray-applied (in an amount of 0.2 mL) from the rotating side surface side of the gasket, and then dried at 100 degrees C for 15 minutes to produce a gasket of the present invention. Thereafter, washing was performed with purified water at 80 degrees C or higher in order to wash off the excess coating liquid on the produced gasket. The coating layer formed on the surface of the core member had an average thickness of about 8 µm. This gasket was used in Example 2.

### (Example 3)

As the silicone-based resin, the following resins were used.

### A2: Silicone-based resin

1) DMS-S14 (product name) (manufactured by Gelest Inc.) containing both terminals silanol polydimethylsiloxane as main component: 25 parts by weight
2) SIP6560.0 (product name) (manufactured by Gelest Inc.) containing methyltrimethoxysilane as main component: 0.1 parts by weight
3) Mixture of SIA0610.0 (product name) (manufactured by Gelest Inc.) containing 3-aminopropyltriethoxysilane as main component with ethanol solution of maleic anhydride: 1 part by weight (resin ratio: 50%)
4) SIG5840.1 (product name) (manufactured by Gelest Inc.) containing 3-glycidoxypropyltrimethoxysilane as main component: 0.5 parts by weight

A silicone-based resin mixture was produced by mixing 10 parts by weight of linear sodium alkylbenzene sulfonate with 100 parts by weight of the silicone-based resin (A2) including 1) to 4) described above. To 66 parts by weight of purified water, 29 parts by weight of the silicone-based resin mixture and 1 part by weight of tin dioctyldilaurate were added to prepare a main agent. To 100 parts by weight of the main agent, 5 parts by weight of the carbodiimide compound (B: mixture of the polyvalent carbodiimide compound and water) used in Example 1 was added, and the resulting mixture was mixed and stirred to prepare a liquid for coating.

Then, a gasket core member produced as described above was heat-treated at 100 degrees C for 15 minutes under a room temperature and normal pressure environment and then rotated (at 300 rpm) around its central axis, and the coating liquid having the above-described composition was spray-applied (in an amount of 0.2 mL) from the rotating side surface side of the gasket, and then dried at 100 degrees C for 15 minutes to produce a gasket of the present invention. Thereafter, washing was performed with purified water at 80 degrees C or higher in order to wash off the excess coating liquid on the produced gasket. The coating layer formed on the surface of the core member had an average thickness of about 8 µm. This gasket was used in Example 3.

### (Example 4)

A liquid for coating was prepared in the same manner as in Example 3 except that 1 part by weight of the carbodiimide compound (B: mixture of the polyvalent carbodiimide compound and water) used in Example 1 was added to 100 parts by weight of the main agent in Example 3 and the resulting mixture was mixed and stirred to prepare a liquid for coating.

Then, a gasket core member produced as described above was heat-treated at 100 degrees C for 15 minutes under a room temperature and normal pressure environment and then rotated (at 300 rpm) around its central axis, and the liquid for coating having the above-described composition was spray-applied (in an amount of 0.2 mL) from the rotating side surface side of the gasket, and then dried at 100 degrees C for 15 minutes to produce a gasket of the present invention. Thereafter, washing was performed with purified water at 80 degrees C or higher in order to wash off the excess coating liquid on the produced gasket. The coating layer formed on the surface of the core member had an average thickness of about 8 µm. This gasket was used in Example 4.

### (Example 5)

As the silicone-based resin, the following resins were used.

### A3: Silicone-based resin

1) YR3204 (product name) (manufactured by Momentive Performance Materials Japan LLC) containing polyalkylphenylsiloxane having silanol group as main component: 25 parts by weight
2) TSL8178 (product name) (manufactured by Momentive Performance Materials Japan LLC) containing phenyltriethoxysilane as main component: 0.1 parts by weight
3) Mixture of TSL8331 (product name) (manufactured by Momentive Performance Materials Japan LLC) containing 3-aminopropyltriethoxysilane as main component with ethanol solution of maleic anhydride: 1 part by weight (resin ratio: 50%)
4) TSL8350 (product name) (manufactured by Momentive Performance Materials Japan LLC) containing 3-glycidoxypropyltrimethoxysilane as main component: 0.5 parts by weight

A silicone-based resin mixture was produced by mixing 10 parts by weight of linear sodium alkylbenzene sulfonate with 100 parts by weight of the silicone-based resin (A3) including 1) to 4) described above. To 66 parts by weight of purified water, 29 parts by weight of the silicone-based resin mixture and 1 part by weight of tin dioctyldilaurate were added to prepare a main agent.

To 100 parts by weight of the main agent, 5 parts by weight of the carbodiimide compound (B: mixture of the polyvalent carbodiimide compound and water) used in Example 1 was added, and the resulting mixture was mixed and stirred to prepare a liquid for coating.

Then, a gasket core member produced as described above was heat-treated at 100 degrees C for 15 minutes under a room temperature and normal pressure environment and then rotated (at 300 rpm) around its central axis, and the coating liquid having the above-described composition was spray-applied (in an amount of 0.2 mL) from the rotating side surface side of the gasket, and then dried at 100 degrees C for 15 minutes to produce a gasket of the present invention. Thereafter, washing was performed with purified water at 80 degrees C or higher in order to wash off the excess coating liquid on the produced gasket. The coating layer formed on the surface of the core member had an average thickness of about 8 µm. This gasket was used in Example 5.

### (Example 6)

As the carbodiimide compound, the following carbodiimide compound different from that in Example 1 was used.

### C: Carbodiimide compound

Aqueous solution of polyvalent carbodiimide [polyvalent carbodiimide compound content: 40 wt%, product name: CARBODILITE V-02-L2L, manufactured by Nisshinbo Chemical Inc., pH: 8 to 11, viscosity (representative value): 70 mPa s, NCN equivalent (chemical formula weight per mol of carbodiimide group): 385, aqueous solution of hydrophilic group-modified polycarbodiimide compound]

A liquid for coating was prepared in the same manner as in Example 1 except that 5 parts by weight of (C: the mixture of the polyvalent carbodiimide compound and water) described above was added to 100 parts by weight of the main agent in Example 1 and the resulting mixture was mixed and stirred to prepare a liquid for coating.

Then, a gasket core member produced as described above was heat-treated at 100 degrees C for 15 minutes under a room temperature and normal pressure environment and then rotated (at 300 rpm) around its central axis, and the liquid for coating having the above-described composition was spray-applied (in an amount of 0.2 mL) from the rotating side surface side of the gasket, and then dried at 100 degrees C for 15 minutes to produce a gasket of the present invention. Thereafter, washing was performed with purified water at 80 degrees C or higher in order to wash off the excess coating liquid on the produced gasket. The coating layer formed on the surface of the core member had an average thickness of about 8 µm. This gasket was used in Example 6.

### (Example 7)

As the carbodiimide compound, the following carbodiimide compound different from those in Examples 1 and 6 was used.

### D: Carbodiimide compound

Aqueous emulsion of polyvalent carbodiimide [polyvalent carbodiimide compound content: 41.3 wt%, product name: CARBODILITE E-05, manufactured by Nisshinbo Chemical Inc., pH: 8 to 11, viscosity (representative value): 100 mPa s, NCN equivalent (chemical formula weight per mol of carbodiimide group): 310, aqueous emulsion of hydrophilic group-modified polycarbodiimide compound]

A liquid for coating was prepared in the same manner as in Example 1 except that 5 parts by weight of (D: the mixture of the polyvalent carbodiimide compound and water) described above was added to 100 parts by weight of the main agent in Example 1 and the resulting mixture was mixed and stirred to prepare a liquid for coating.

Then, a gasket core member produced as described above was heat-treated at 100 degrees C for 15 minutes under a room temperature and normal pressure environment and then rotated (at 300 rpm) around its central axis, and the liquid for coating having the above-described composition was spray-applied (in an amount of 0.2 mL) from the rotating side surface side of the gasket, and then dried at 100 degrees C for 15 minutes to produce a gasket of the present invention. Thereafter, washing was performed with purified water at 80 degrees C or higher in order to wash off the excess coating liquid on the produced gasket. The coating layer formed on the surface of the core member had an average thickness of about 8 µm. This gasket was used in Example 7.

### (Comparative Example 1)

A silicone-based resin mixture (A) was produced by mixing 10 parts by weight of linear sodium alkylbenzene sulfonate with 100 parts by weight of the silicone-based resin (A1) in Example 1. To 66 parts by weight of purified water, 29 parts by weight of the silicone-based resin mixture (A) and 1 part by weight of tin dioctyldilaurate were added to prepare a liquid for coating.

Then, a gasket core member produced as described above was heat-treated at 100 degrees C for 15 minutes under a room temperature and normal pressure environment and then rotated (at 300 rpm) around its central axis, and the coating liquid having the above-described composition was spray-applied (in an amount of 0.2 mL) from the rotating side surface side of the gasket, and then dried at 100 degrees C for 15 minutes to produce a gasket of the present invention. Thereafter, washing was performed with purified water at 80 degrees C or higher in order to wash off the excess coating liquid on the produced gasket. The coating layer formed on the surface of the core member had an average thickness of about 8 µm. This gasket was used in Comparative Example 1.

### (Experiment 1: Film Strength Test)

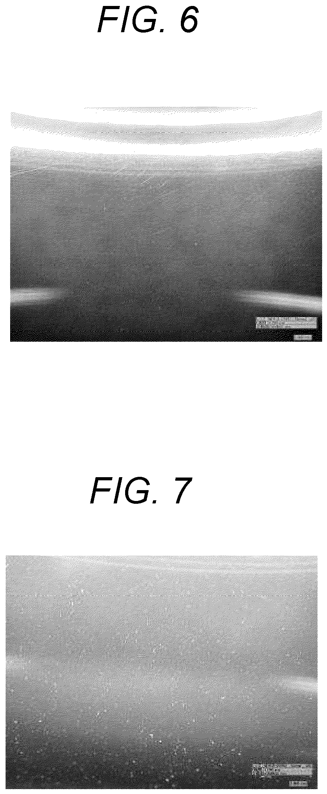

A plastic flat plate including a cyclic polyolefin (trade name: ZEONEX, manufactured by Zeon Corporation) as a forming material was prepared. The gasket of each of Examples 1 to 7 and Comparative Example 1 was brought into sliding contact with the surface of the plastic flat plate by 90 mm at a speed of 100 mm/min in the horizontal direction with a force of 10 N while the side surface of the gasket having the coating layer was pressed in the vertical direction. The appearance of the plastic flat plate after completion of the above test was confirmed. In the gasket of each of Examples 1 to 7, an adhered matter was not confirmed on the plastic flat plate, as shown in FIG. 6. In the gasket of Comparative Example 1, an adhered matter considered to be a matter peeled off from the coating layer was confirmed on the plastic flat plate, as shown in FIG. 7.

### (Experiment 2: Sliding Resistance Measurement Test)

An outer cylinder, for use in a syringe, having the shape shown in FIG. 5 was produced by injection molding using a cyclic polyolefin (trade name: ZEONEX, manufactured by Zeon Corporation) as a material for formation of the outer cylinder for use in a 100 mL syringe. The outer cylinder for use in a syringe had a cylindrical part having an inner diameter of 32 mm and a length of 154 mm. Furthermore, a plunger having the shape shown in FIG. 5 was produced by injection molding using polypropylene (manufactured by Japan Polychem Corporation) as a material for formation of the plunger.

Then, the outer cylinder for use in a syringe, the gasket of each of Examples 1 to 7 and Comparative Example 1, and the plunger were assembled to produce a syringe.

The sliding resistance value of the syringe was measured with an autograph (model name: EZ-Test, company name: SHIMADZU CORPORATION). Specifically, the sliding resistance value (N) was measured when the distal end of the syringe and the rear end of the plunger were fixed to the measurement object fixing portion of the autograph and the plunger was lowered by 60 mm at a speed of 100 mm/min. As a result, the results shown in Table 1 were obtained.

**(Table 1)**

| Sliding resistance value (N) | |
|---|---|
| Example 1 | 100 N |
| Example 6 | 110 N |
| Example 7 | 105 N |
| Comparative Example 1 | 105 N |

The syringes with the gaskets of Examples 1, 6, and 7 and Comparative Example 1 had similar initial sliding resistance values and maximum sliding resistance values. The difference between the initial sliding resistance value and the maximum sliding resistance value is small, and more than a set amount of the drug solution is less likely to be forced out when the plunger starts to be pushed, so that the drug solution can be safely and accurately discharged. In Examples 2 to 5, the sliding performance was also almost the same as in Examples 1, 6, and 7.

### (Experiment 3: Pressure Test Specified in Standards for Sterile Injection Syringes)

An outer cylinder, for use in a syringe, having the shape shown in FIG. 5 was produced by injection molding using a cyclic polyolefin (trade name: ZEONEX, manufactured by Zeon Corporation) as a material for formation of the outer cylinder for use in a 100 mL syringe. The outer cylinder for use in a syringe had a cylindrical part having an inner diameter of 32 mm and a length of 154 mm. Furthermore, a plunger having the shape shown in FIG. 5 was produced by injection molding using polypropylene (manufactured by Japan Polychem Corporation) as a material for formation of the plunger.

Then, the outer cylinder for use in a syringe, the gasket of each of Examples 1 to 7 and Comparative Example 1, and the plunger were assembled to produce a syringe.

The test was performed that is specified as a pressure test in the standards for sterile injection syringes (notified on Dec. 11, 1998 by director of pharmaceutical and medical safety bureau in 1079 issue of pharmaceutical development) specifying the items required for a sterilized plastic injection syringe that can be immediately used as it is and is to be discarded after one-time use. The gaskets of Examples 1 to 7 and Comparative Example 1 conformed.

The gasket for use in a syringe of the present invention is formed to be in liquid-tight and slidable contact with the inside of a plastic outer cylinder of a syringe, the gasket includes: a gasket body including an elastic body; and a coating layer provided at least at a part in contact with the syringe, the coating layer is an elastic cured coating layer containing a silicone-based resin as a main component and containing a carbodiimide compound together with a silane coupling agent, and the silicone-based resin includes a condensate of a reactive silicone having a silanol group at both terminals and has a siloxane bond derived from the silanol group.

Therefore, the gasket has stable sliding performance without applying a lubricant to the sliding surface. Furthermore, the coating layer has elasticity and sufficient strength, and therefore has an extremely low possibility of peeling off and maintains good sliding performance.

The gasket for use in a syringe of the present invention is as follows. (1) A gasket for use in a syringe, the gasket formed to be in liquid-tight and slidable contact with an inside of a plastic outer cylinder of a syringe, the gasket including: a gasket body including an elastic body; and a coating layer provided at least at a part in contact with the syringe, the coating layer being an elastic cured coating layer containing a silicone-based resin as a main component and containing a carbodiimide compound together with a silane coupling agent, the silicone-based resin that includes a condensate of a reactive silicone having a silanol group at both terminals and has a siloxane bond derived from the silanol group.

The gasket for use in a syringe of the present invention is formed to be in liquid-tight and slidable contact with the inside of a plastic outer cylinder of a syringe, the gasket includes: a gasket body including an elastic body; and a coating layer provided at least at a part in contact with the syringe, the coating layer is an elastic cured coating layer containing a silicone-based resin as a main component and containing a carbodiimide compound together with a silane coupling agent, and the silicone-based resin includes a condensate of a reactive silicone having a silanol group at both terminals and has a siloxane bond derived from the silanol group.

Therefore, the gasket has stable sliding performance without applying a lubricant to the sliding surface. Furthermore, the coating layer has elasticity and sufficient strength, and therefore has an extremely low possibility of peeling off and maintains good sliding performance.

Furthermore, the above-described embodiment may be as follows.
(2) The gasket for use in a syringe according to (1) described above, wherein the carbodiimide compound is a carbodiimide having two or more carbodiimide groups.
(3) The gasket for use in a syringe according to (1) or (2) described above, wherein the carbodiimide compound is a polycarbodiimide.
(4) The gasket for use in a syringe according to any one of (1) to (3) described above, wherein the silicone-based resin or the silane coupling agent has a functional group that reacts with a carbodiimide group of the carbodiimide compound.
(5) The gasket for use in a syringe according to (4) described above, wherein the functional group that reacts with a carbodiimide group is at least one selected from a carboxyl group, a hydroxyl group, an amino group, an amide group, a sulfonate group, and a phosphate group.
(6) The gasket for use in a syringe according to any one of (1) to (5) described above, wherein the silane coupling agent is at least one selected from an alkylalkoxysilane, a phenylalkoxysilane, an aminoalkylalkoxysilane, and a glycidoxyalkylalkoxysilane.
(7) The gasket for use in a syringe according to any one of (1) to (5) described above, wherein the silane coupling agent contains an alkylalkoxysilane or a phenylalkoxysilane as a first silane coupling agent, and further contains an aminoalkylalkoxysilane or a glycidoxyalkylalkoxysilane as a second silane coupling agent.
(8) The gasket for use in a syringe according to any one of (1) to (5) described above, wherein the silane coupling agent contains an alkylalkoxysilane or a phenylalkoxysilane as a first silane coupling agent, and further contains an aminoalkylalkoxysilane as a second silane coupling agent and a glycidoxyalkylalkoxysilane as a third silane coupling agent.
(9) The gasket for use in a syringe according to any one of (1) to (8) described above, wherein the elastic cured coating layer is a thermosetting elastic cured coating layer.
(10) The gasket for use in a syringe according to any one of (1) to (9) described above, wherein the coating layer has a thickness of 1 to 30 µm.

Furthermore, the syringe of the present invention is as follows. (11) A syringe including: a plastic outer cylinder; the gasket described in any one of (1) to (10) above slidably accommodated in the plastic outer cylinder; and a plunger attached to or attachable to the gasket.

Furthermore, the above-described embodiment may be as follows. (12) The syringe according to (11) described above, filled with a drug solution.

## Claims

1. A gasket for use in a syringe, the gasket formed to be in liquid-tight and slidable contact with an inside of a plastic outer cylinder of a syringe, the gasket comprising: a gasket body including an elastic body; and a coating layer provided at least at a part in contact with the syringe, the coating layer being an elastic cured coating layer containing a silicone-based resin as a main component and containing a carbodiimide compound together with a silane coupling agent, the silicone-based resin that includes a condensate of a reactive silicone having a silanol group at both terminals and has a siloxane bond derived from the silanol group.

2. The gasket for use in a syringe according to claim 1, wherein the carbodiimide compound is a carbodiimide having two or more carbodiimide groups.

3. The gasket for use in a syringe according to claim 1 or 2, wherein the carbodiimide compound is a polycarbodiimide.

4. The gasket for use in a syringe according to any one of claims 1 to 3, wherein the silicone-based resin or the silane coupling agent has a functional group that reacts with a carbodiimide group of the carbodiimide compound.

5. The gasket for use in a syringe according to claim 4, wherein the functional group that reacts with a carbodiimide group is at least one selected from a carboxyl group, a hydroxyl group, an amino group, an amide group, a sulfonate group, and a phosphate group.

6. The gasket for use in a syringe according to any one of claims 1 to 5, wherein the silane coupling agent is at least one selected from an alkylalkoxysilane, a phenylalkoxysilane, an aminoalkylalkoxysilane, and a glycidoxyalkylalkoxysilane.

7. The gasket for use in a syringe according to any one of claims 1 to 5, wherein the silane coupling agent contains an alkylalkoxysilane or a phenylalkoxysilane as a first silane coupling agent, and further contains an aminoalkylalkoxysilane or a glycidoxyalkylalkoxysilane as a second silane coupling agent.

8. The gasket for use in a syringe according to any one of claims 1 to 5, wherein the silane coupling agent contains an alkylalkoxysilane or a phenylalkoxysilane as a first silane coupling agent, and further contains an aminoalkylalkoxysilane as a second silane coupling agent and a glycidoxyalkylalkoxysilane as a third silane coupling agent.

9. The gasket for use in a syringe according to any one of claims 1 to 8, wherein the elastic cured coating layer is a thermosetting elastic cured coating layer.

10. The gasket for use in a syringe according to any one of claims 1 to 9, wherein the coating layer has a thickness of 1 to 30 µm.

11. A syringe comprising: a plastic outer cylinder; the gasket for use in a syringe according to any one of claims 1 to 10, the gasket slidably accommodated in the plastic outer cylinder; and a plunger attached to or attachable to the gasket.

12. The syringe according to claim 11, filled with a drug solution.
